# EUROPEAN PATENT SPECIFICATION

(11) **EP 2 178 458 B1**
(45) Date of publication and mention of the grant of the patent: **19.11.2014**
(21) Application number: 08757920.7
(22) Date of filing: 19.06.2008
(51) Int. Cl.: A61L 27/38, A61L 27/56, A61L 27/24

(54) **PROCEDURE FOR THE MANUFACTURE OF A STRUCTURED MULTILAYER MEMBRANE, THE STRUCTURED MULTILAYER MEMBRANE AND ITS USES**
VERFAHREN ZUR HERSTELLUNG EINER STRUKTURIERTEN MEHRLAGIGEN MEMBRAN, STRUKTURIERTE MEHRLAGIGE MEMBRAN UND VERWENDUNGEN DAFÜR
PROCÉDÉ DE FABRICATION D'UNE MEMBRANE MULTICOUCHE STRUCTURÉE, MEMBRANE MULTICOUCHE STRUCTURÉE ET SES UTILISATIONS

(30) Priority: 21.06.2007 CZ 20070424
(43) Date of publication of application: 28.04.2010
(73) Proprietor: Hypro Otrokovice S.R.O., 75602 Otrokovice (CZ)
(72) Inventor: GALATIK, Antonin, CZ-76502 Otrokovice (CZ)
(74) Representative: Kreizlova, Dana
(86) International application number: PCT/CZ2008/000071
(87) International publication number: WO 2008/154888

(56) References cited:
- WO-A-99/47188
- WO-A1-99/19005
- US-A1- 2002 013 627

## Description

### Scope of technology

The invention is a structured multilayer membrane and procedure for controlled cartilage, tissue or bone regeneration or repair in vivo and a procedure for the manufacture of the said structured multilayer membrane.

### Present status of technology

It is now generally accepted that reconstruction of tissue requires the provision of a matrix to serve as a guide orienting and, on the other hand, limiting the direction of cell growth during the healing process. In a procedure described in international patent application WO-A-96/25961 , a structurally homogeneous single-layer matrix consisting of natural membranes formed by collagen Il is used. Such membranes, however, fail to regenerate cartilage tissues to a sufficient extent. Therefore, a multilayer membrane consisting of a membrane made of collagen Il and connected by sewing to another layer consisting of a porous matrix made of collagen I and allowing cell ingrowth, was devised as described in patent application WO-A-96/24310. A next improvement was brought about by a procedure described by patent application WO 1999/019005, where instead of by sewing, a reasonably strong bonding between the layers is achieved through adhesion of the collagen gel, which can be enhanced by the effect of chemical or physico-chemical cross-linking. In order to reduce pyrogenicity of the collagen implants, CS 297 205 patent describes a procedure using atelocollagen, a modification of collagen from which the terminal telopeptides containing antigenic determinants have been removed (partly at least). An extension of the time of membrane absorption, accompanied by a controlled release of pharmaceutically active components (if present) via metallo-complex networking and collagen grafting with hyaluronan is the core of a procedure described by Czech patent application PV 2006-409.

WO 99/47188 A1 discloses a biopolymer matt composite for tissue repair and tissue reconstruction as well as methods for making and using said biopolymer matt. The matt composite comprises different layers of collagen I which is freed from telopeptides. The porosity of the collagen three-dimensional matrix can be tailored by freeze-drying process. Substances such as glycosaminoglycans, chondrocytes and growth factors are added to the biopolymer.

US2002/0013627 A1 discloses a method for promoting regeneration of surface cartilage of a joint with a patch covering the area to be treated, said patch comprising a sheet of collagen membrane material composed of at least one barrier layer and a matrix layer having an open sponge-like texture. The open sponge-like structure is achieved by freeze-drying. The collagen is freed from telopeptides and the matrix is impregnated with glycosaminoglycans, chondrocytes and other substances such as transforming growth factors.

We found that cartilage and/or bone tissue can be also reconstructed by using single-component membranes whose structure comprises horizontally arranged layers with different material densities. The density of a layer is determined by the number and size of pores or capillary channels they contain. While a layer possessing a dense structure acts as a barrier preventing the penetration of chondrocytes and other cells, another membrane layer possesses an open and porous structure allowing cell ingrowth. The transition between the two structures can be smooth, without any sharp boundary between them.

### Subject of the invention

The invention provides a structured multilayer membrane applicable to surgical reconstruction of dental, bone, cartilage, or ligament tissue., The membrane consists of at least one closed for cells impermeable layer - barrier - and at least one porous for the migration of the cells open layer and the whole membrane is made of the pure animal collagen of type I, from which telopeptides have been removed (partly at least) and which can be cross-linked.

Also it can contain pharmacologically active substances, particularly glycosaminoglycans, (ii) can contain at least one structural layer closed to cell ingrowth, and (iii) can contain at least one porous structural layer open to cell ingrowth.

The procedure consists of steps where a homogeneous porous membrane is created by freeze-drying of plates of frozen collagen gel and the membrane is then wetted by controlled application of a mixture of water and additives, if any, to one or both surfaces and the material is allowed to hydrate for a period of time, conveniently at a reduced temperature. The thickness of the wetted membranes is then modified as appropriate by application of pressure, and the structure obtained is stabilized by rapid freezing. A non-porous structure of the membrane layer is formed during a process where final drying is conducted at a reduced pressure, the temperature of the plates being allowed to increase to above the freezing point within a pre-tested phase. The thickness and density of the non-porous structure in the membranes can be controlled by varying the length of the period of drying at temperatures above the freezing point and the total water content of the membrane.

Membranes so prepared are not prone to decomposition into the layers during the healing process and exhibit improved apyrogenicity, owing to the fact that a collagen type - conveniently atelocollagen modified by lowering of its telopeptide content - is used, whereby the total amount of antigenic determinants in the implanted membranes is reduced.

A membrane so prepared constitutes a suitable medium for the ingrowth of natural chondrocytes and thus to the regeneration of the cartilage. The recovery process can be additionally promoted by impregnating the membrane with chondrocytes or other pharmacologically active substances, either prior to its implantation or after implantation, by the injection route.

The membrane collagen material can conveniently consist of pure non-denatured and insoluble collagen and can be prepared, for instance, by procedures described in patent document CS 276 891 or in Czech patent application PV 2006-409.

### Examples of application of the invention

### Example 1

Connective tissue of bovine tendons from young cattle is mechanically freed from fat and from the connective tissue capsule, ground, rinsed under running water, and extracted triply with a 10% sodium chloride solution for 24 hours and then at least triply with a half-saturated solution of calcium hydroxide for 24 hours until its non-collagen protein content has decreased to below 1%. Subsequently, the material is neutralized with acetic acid, washed with water, dehydrated with ethanol, and freed from residual fat by extraction with benzine. The resulting fibrous material is dispersed in demineralized water, freed from telopeptides by an enzymatic process, washed with demineralized water, and dissolved by acidification to pH 3,5. A gel-like suspension containing 10 g of atelocollagen in a litre is thus obtained.

A homogeneous porous membrane is then formed by freeze-drying of collagen gel plates obtained by freezing in flat beds at -15°C. The membrane is wetted by controlled application of water to one or both surfaces so that the amount of water does not exceed 20% of the membrane weight, and the material is allowed to hydrate at 0°C to 5°C for 5 hours. Pressure is applied to a compression plate to attain a suitable thickness of the wet membranes, usually between 3 mm and 0.1 mm, and the structure achieved is stabilized by rapid freezing. The frozen membranes are dried at a reduced pressure to the final state, the temperature of the plates being allowed to increase to 3°C - 5°C during a pre-tested phase.

### Example 2

A membrane is prepared as in Example 1, only an aqueous solution containing 0.15% hyaluronan, 0,001% magnesium chloride, and 5 mg/l joint cartilage chondrocytes is used in place of water in the wetting step. The ensuing procedure is identical with that described in Example 1.

## Claims

1. A structured multilayer membrane applicable to surgical reconstruction of dental, bone, cartilage, or ligament tissue, **characterized by that** the membrane contains at least one structural layer closed to cell ingrowth and contains at least one porous structural layer open to cell ingrowth and the whole membrane is made of the pure animal collagen of type I.

2. A structured multilayer membrane according to the claim 1, **characterized by that** the collagen has his polypeptidic chains freed from terminal telopeptides, partly at least.

3. A structured multilayer membrane according to the claim 1, **characterized by that** the collagen macromolecules are cross-linked and they contain glycosaminoglycan which is bonded to them by metallo-complex bonding.

4. A structured multilayer membrane according to the claim 1, **characterized by that** the membrane contains chondrocytes.

5. A structured multilayer membrane according to the claim 1, **characterized by that** the membrane contains glycosaminoglycan.

6. A structured multilayer membrane according to the claim 1, **characterized by that** the membrane contains one of more ingredients from the group involving chondronectin, laminin, fibronectin, calcium alginate, anchorin, bacteriostatics, pharmacologically active substances, growth factors and/or morphogenetic bone factors.

7. A method of the manufacturing of a structured multilayer membrane according to the claim 1, **characterized by that** the suspension of the pure animal collagen of type I accommodated in a flatbed is frozen into plates, which are then vacuum-dried at a temperature lower than their freezing temperature, and the obtained sponge-like membranes are wetted by a limited amount of water which is less than amount sufficient for the hydration of the whole membrane thickness and the wetted membrane is conveniently allowed to rest at a reduced temperature of 0°C to 10°C so as to enable the moisture to equilibrate inside the structure, then mechanical pressure is applied in order to modify the thickness of the membrane as appropriate, one outer side is differentiated from the other by applying a tread pattern or dye, the plate is re-frozen and dried at a reduced pressure, the drying procedure being such that at a later stage the temperature of the material is gradually increased to that above the melting point, whereby a closed structure is attained by drying in that part of the membrane which is still wet.

## Patentansprüche

1. Ein strukturiertes Mehrschichtmembran für die chirurgische Rekonstruktion von Zahn, Knochen, Knorpel, oder Ligament Gewebe, **dadurch gekennzeichnet, dass** die Membrane mindestens auf die Strukturschicht auf das Einwachsen von Zellen geschlossen sind und enthalten wenigstens eine poröse Strukturschicht, um das Einwachsen von Zellen und das gesamte Membran wird vom reinen Tier-Kollagen vom Typ I gemacht.

2. Ein strukturiertes Mehrschichtmembran nach dem Anspruch 1, **dadurch gekennzeichnet, dass** das Kollagen seine Polypeptid-Ketten vom Anschluß der Telopeptide befreit, teilweise zumindest.

3. Ein strukturiertes Mehrschichtmembran nach dem Anspruch 1, **dadurch gekennzeichnet, dass** die Makromoleküle Kollagen vernetzt sind und Glycosaminoglycan, das mit ihnen von Metallo-Komplexbindung gebunden ist, enthalten.

4. Ein strukturiertes Mehrschichtmembran nach dem Anspruch 1, **dadurch gekennzeichnet, dass** die Membran Chondrozyten enthält.

5. Ein strukturiertes Mehrschichtmembran nach dem Anspruch 1, **dadurch gekennzeichnet, dass** die Membran Glycosaminoglycan enthält.

6. Ein strukturiertes Mehrschichtmembran nach dem Anspruch 1, **dadurch gekennzeichnet, dass** die Membran eine oder mehrere Zutaten aus der Gruppe mit Chondronectin, Iamin, Fibronektin, Calciumalginat, Anchorin, Bakteriostatika, pharmakologisch aktiven Substanzen, Wachstumsfaktoren und/oder Knochenmorphogenen Faktoren enthält.

7. Verfahren zur Herstellung eines strukturierten mehrschichtigen Membran nach dem Anspruch 1, **dadurch gekennzeichnet, dass** die Suspension des reinen Tier Kollagen vom Typ I in einem Flachbett untergebracht ist, in Platten, die dann im Vakuum getrocknet, bei einer Temperatur niedriger als die Gefriertemperatur gefroren und die erhaltenen schwammartigen Membranen werden durch eine begrenzte Menge an Wasser, die weniger als ausreichende Menge für die Hydratation der gesamten Membrandicke und die benetzte Membran liegt ruhen gelassen bei einer reduzierten Temperatur von 0°C bis 10°C benetzt um so die Feuchtigkeit in das Innere der Struktur zu äquilibrieren, dann wird ein mechanischer Druck aufgelegt, um die Dicke der Membran nach Bedarf modifiziert aufgebracht wird, eine Außenseite von der anderen durch Aufbringen eines Laufflächenmusters oder der Farbstoff unterscheiden, wird die Platte wieder eingefroren und getrocknet, bei einem reduzierten Druck, der Trocknungsvorgang derart ist, daß zu einem späteren Zeitpunkt die Temperatur des Materials allmählich auf die über dem Schmelzpunkt erhöht wird, wodurch eine geschlossene Struktur durch Trocknung in dem Teil der Membran, die noch feucht ist erreicht wird .

## Revendications

1. Membrane multicouche structurée applicable à la reconstruction chirurgicale dentaire, osseuse, cartilagineuse ou des tissus ligamentaires, **caractérisée par le fait que** la membrane contient au moins une couche structurale hostile à l'envahissement cellulaire et contient au moins une couche structurale poreuse propice à l'envahissement cellulaire. L'ensemble de la membrane est constitué de collagène animal pur de type I.

2. Membrane multicouche structurée selon la revendication 1, **dans lequel** le fait que les chaînes polypeptidiques du collagène sont exemptes de télopeptides terminaux, au moins partiellement.

3. Membrane multicouche structurée selon la revendication 1, **dans lequel** le fait que les macromolécules de collagène sont réticulées et contiennent des glycosaminoglycanes qui leur sont reliés par une liaison métallo-complexe.

4. Membrane multicouche structurée selon la revendication 1, **dans lequel** le fait que la membrane contient des chondrocytes.

5. Membrane multicouche structurée selon la revendication 1, **dans lequel** le fait que la membrane contient des glycosaminoglycanes.

6. Membrane multicouche structurée selon la revendication 1, **dans lequel** le fait que la membrane contient un ou plusieurs ingrédients du groupe incluant la chondronectine, la laminine, la fibronectine, l'alginate de calcium, l'anchorin, les bactériostatiques, les substances pharmacologiquement actives, les facteurs de croissance ou les facteurs morphogénétiques osseux.

7. Procédé de fabrication d'une membrane multicouche structurée selon la revendication 1, **dans lequel** le fait que la suspension du collagène animal pur de type I logé dans un flatbet est congelée dans les plaques, lesquelles sont ensuite séchées sous vide à une température inférieure à la température de congélation; les membranes spongieuses obtenues sont ensuite imbibées d'une quantité d'eau limitée, inférieure à la quantité suffisante pour hydrater totalement l'épaisseur de la membrane, la membrane mouillée est ensuite laissée au repos à une température réduite de 0 °C à 10 °C, de manière à permettre à l'humidité de s'équilibrer à l'intérieur de la structure; puis, une pression mécanique est exercée afin de modifier l'épaisseur de la membrane comme il convient, les faces extérieures étant différenciées l'une de l'autre par application d'un motif à nervures ou d'un colorant; la plaque est ensuite recongelée et séchée à une pression réduite, la procédure de séchage implique, à un stade ultérieur, l'augmentation progressive de la température de la matière pour atteindre une température supérieure au point de fusion, afin d'obtenir une structure fermée par séchage de la partie de la membrane encore humide.
